# FASCICULE DE BREVET EUROPEEN

(11) **EP 3 441 103 B1**
(45) Date de publication et mention de la délivrance du brevet: **03.06.2020**
(21) Numéro de dépôt: 18168307.9
(22) Date de dépôt: 19.04.2018
(51) Int. Cl.: A61M 16/16, H05B 3/04, H05B 3/14, A61M 16/10

(54) **RÉSERVOIR À EAU POUR HUMIDIFICATEUR DE GAZ À PLAQUE CHAUFFANTE COLLÉE**
WASSERTANK FÜR GAS-BEFEUCHTER MIT GEKLEBTER HEIZPLATTE
WATER TANK FOR GAS HUMIDIFIER WITH BONDED HEATING PLATE

(30) Priorité: 08.08.2017 FR 1757575
(43) Date de publication de la demande: 13.02.2019
(73) Titulaire: Air Liquide Medical Systems, 92160 Antony (FR)
(72) Inventeur: LEBATTEUR, Nicolas, 91220 BRETIGNY SUR ORGE (FR); SCHIFFANO, Nicolas, 33400 TALENCE (FR)
(74) Mandataire: Pittis, Olivier

(56) Documents cités:
- EP-A1- 2 848 277
- WO-A1-00/21602
- FR-A3- 3 008 319

## Description

L'invention porte sur un réservoir à eau pour humidificateur de gaz comprenant une plaque métallique chauffante collée au fond de la cuve à eau, ainsi que sur un humidificateur équipé d'un tel réservoir.

Les appareils médicaux d'assistance respiratoire ou ventilatoire, encore appelés ventilateurs médicaux, sont couramment utilisés pour administrer des gaz respiratoires, tel de l'air, aux personnes souffrant de problèmes respiratoires. Le gaz respiratoire est généralement humidifié avant son administration aux patients au moyen d'un dispositif d'humidification de gaz.

Un dispositif d'humidification de gaz chauffant, encore appelé « humidificateur de gaz », comprend un réservoir amovible renfermant une cuve destinée à contenir un volume d'eau à chauffer. Le réservoir est configuré pour permettre le passage d'un débit d'air entre un orifice d'entrée et un orifice de sortie. Son fond comporte une plaque métallique chauffante destiné à transmettre à l'eau, de la chaleur, c'est-à-dire des calories, provenant d'un boitier chauffant, dans lequel vient se loger le réservoir. Le fond du boitier comporte un élément chauffant relié à ou en contact avec une autre plaque métallique dont le rôle est de transmettre la chaleur produite par l'élément chauffant du boitier à la plaque métallique du réservoir.

Pendant le fonctionnement de l'humidificateur, l'air provenant d'un ventilateur médical, entre dans le réservoir de l'humidificateur, s'y réchauffe et s'y charge en humidité grâce à l'eau qui est chauffée dans le réservoir de l'humidificateur.

L'air chaud humidifié est ensuite acheminé jusqu'aux voies respiratoires du patient devant l'inhaler via par exemple un conduit de gaz flexible alimentant une interface respiratoire, tel un masque respiratoire ou analogue.

Un problème récurrent concerne l'intégration de la plaque métallique chauffante dans le fond de la cuve de l'humidificateur, c'est-à-dire sa fixation de manière solidaire et étanche dans le fond de la cuve de l'humidificateur.

En effet, les cuves actuelles présentent souvent des défauts d'étanchéité ou leurs procédés de fabrication mettent en œuvre des étapes compliquées comme la mise en place d'un joint avec une plaque vissée dans un plastique technique résistant.

Par ailleurs, WO-A-00/21602 propose un procédé pour faire adhérer un dome en plastique à une base métallique, notamment utilisables en tant que chambre d'humidification d'un appareil d'assistance respiratoire. La fixation et l'étanchéité sont obtenues au moyen d'un adhésif acrylique à base d'acrylate et d'acrylamide modifié.

Le problème est donc de proposer un réservoir à eau pour humidificateur de gaz qui présente une solidarisation efficace de la plaque métallique chauffante dans le fond de la cuve, ainsi qu'une bonne étanchéité fluidique entre eux, un encombrement réduit ou maitrisé, l'utilisation d'un plastique non technique, une résistance aux déformations liées à la chaleur du fait d'une dilatation différentielle entre la plaque métallique et le plastique de la cuve du réservoir, et qui puisse être obtenue par une méthode simple de fabrication, par exemple ne nécessitant pas l'utilisation d'un outillage d'injection supplémentaire ou l'ajout d'un joint d'étanchéité encombrant, qui nécessiterait en outre une opération manuelle et la présence d'un mécanisme de tenue.

La solution de la présente invention porte sur un réservoir à eau pour humidificateur de gaz comprenant :
- une cuve comprenant une paroi périphérique et un fond délimitant un volume interne destiné à recevoir de l'eau à chauffer, et
- une plaque métallique chauffante agencée dans le fond de la cuve,
caractérisé en ce que la plaque métallique chauffante est fixée de manière étanche dans le fond de la cuve au moyen d'au moins une première colle déposée entre la plaque chauffante et le fond de la cuve, et une deuxième colle, différentes l'une de l'autre, où :
- la première colle est choisie parmi les colles de type néoprène, nitrile, polyuréthane, silicone, acrylique, anaérobique, cyanoacrylate, époxy, inorganique, phénolique, polyester ou polysulfure, et est déposée entre le fond de la cuve et la plaque métallique pour assurer une étanchéité entre le fond de la cuve et la plaque métallique, et garantir leur solidarisation,
- la deuxième colle est une colle capillaire déposée au-dessus de la première colle et remplissant les interstices entre la plaque métallique, la première colle et la cuve du réservoir.

Dans le cadre de l'invention, ce sont les colles utilisées qui permettent de garantir non seulement la fixation/solidarisation de la plaque métallique au fond de cuve mais aussi l'étanchéité entre le rebord périphérique de la plaque métallique et le fond de cuve.

Selon le cas, le réservoir à eau de l'invention peut comprendre l'une ou plusieurs des caractéristiques techniques suivantes :
- la deuxième colle est une colle capillaire de type colle UV à base acrylate ou à base époxy dont le durcissement s'opère en réponse à un éclairage par rayonnement ultraviolet.
- la paroi périphérique est en plastique, de préférence en polypropylène ou analogue.
- la plaque métallique chauffante est en acier inoxydable, en aluminium ou en alliage d'aluminium, en cuivre ou en laiton.
- le fond de la cuve comprenant un épaulement comprenant une rainure périphérique destinée à recevoir au moins une partie de la colle de manière fixer la plaque métallique chauffante audit épaulement, ladite plaque métallique chauffante étant positionnée sur l'épaulement et hors de la rainure périphérique.
- le fond de la cuve comprend deux portions de paroi parallèles l'une à l'autre prenant en sandwich le bord périphérique de ladite plaque métallique chauffante.
- les deux portions de paroi sont moulées autour du bord périphérique de ladite plaque métallique chauffante, c'est-à-dire son pourtour.
- la plaque chauffante est de forme polygonale, de préférence rectangulaire.
- la cuve est de forme générale parallélépipédique rectangle.
- alternativement, la cuve est de forme générale cylindrique ou analogue.
- la cuve comprend un couvercle amovible fermant l'extrémité supérieure de la cuve.
- la cuve comprend un couvercle amovible fixée à la paroi périphérique de la cuve.
- la cuve comprend un couvercle comprenant un orifice d'entrée de gaz et un orifice de sortie de gaz.

L'invention concerne aussi un humidificateur de gaz, typiquement d'air, comprenant un réservoir à eau selon l'invention, de préférence un réservoir amovible.

Par ailleurs, l'invention porte aussi sur une installation de ventilation comprenant un ventilateur médical alimentant un humidificateur de gaz selon l'invention en gaz respiratoire, en particulier de l'air ou de l'air enrichi en oxygène.

L'invention va maintenant être mieux comprise grâce à la description détaillée suivante, faite à titre illustratif mais non limitatif, en référence aux figures annexées parmi lesquelles :
- la Figure 1 schématise un ensemble ventilateur/humidificateur de gaz médical incorporant un réservoir à eau selon l'invention,
- les Figures 2 à 4 schématisent un premier mode de réalisation d'un réservoir à eau selon l'invention, et
- les Figures 5 et 6 schématisent un second mode de réalisation d'un réservoir à eau selon l'invention.

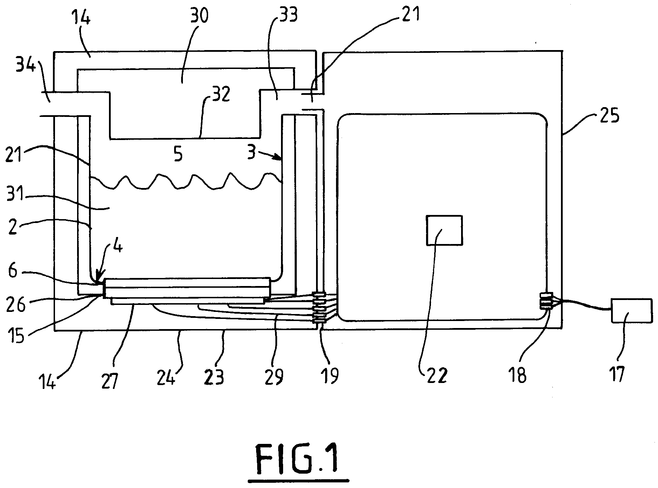

La Figure 1 schématise un mode de réalisation d'un ensemble ventilateur médical/humidificateur de gaz médical comprenant un ventilateur médical 25, c'est-à-dire un appareil de fourniture de gaz d'assistance respiratoire, et un humidificateur de gaz 24 en communication fluidique (en 21) et électrique (en 19) l'un avec l'autre.

Plus précisément, le ventilateur médical 25 comprend des moyens de fourniture de gaz, telle une micro-soufflante électrique, encore appelée « turbine » ou « compresseur », permettant de fournir un flux gazeux, par exemple de l'air, à l'humidificateur 24, et des moyens de commande 20, 22, à savoir une carte électronique 20 à (micro)processeur 22. Le flux de gaz délivré par les moyens de fourniture de gaz du ventilateur médical 25 est acheminé, via un circuit de gaz interne du ventilateur comprenant un ou plusieurs conduits de gaz ou analogue, jusqu'à une sortie de gaz 21, tel un connecteur pneumatique, assurant une communication fluidique entre le ventilateur médical 25 et l'humidificateur 24 de gaz.

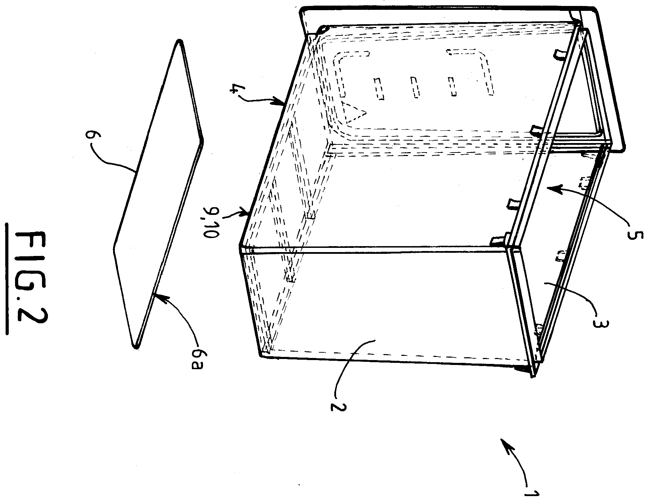

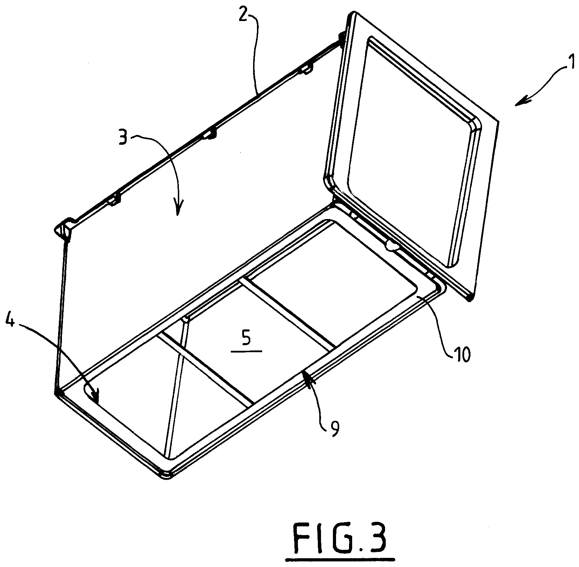

Par ailleurs, l'humidificateur de gaz 24 comprend un boitier 14 délimité par une carcasse externe 23 et incluant un logement 30 à réservoir destiné à recevoir un réservoir à eau 1 amovible, tel celui des Figures 2 à 6, comprenant un bac ou cuve 2 destiné à contenir de l'eau 31 servant à humidifier le flux de gaz provenant du ventilateur 25.

La cuve ou bac 2 à eau comprend un couvercle 32, de préférence amovible, c'est-à-dire détachable, fermant le haut de la cuve 2. Le couvercle 32 agencé sur la cuve 2 est rendu solidaire de la paroi périphérique 3 de la cuve 2, grâce à un système de fixation adapté, par exemple un système par emboitement ou analogue. Le système de fixation assure aussi une étanchéité fluidique entre le rebord périphérique inférieur du couvercle 32 et le rebord périphérique supérieur de la cuve 2. A cette fin, le rebord périphérique inférieur du couvercle 32 et le rebord périphérique supérieur de la cuve 2 viennent préférentiellement s'emboiter l'un dans l'autre.

La cuve 2 du réservoir à eau 1 comprend une entrée 33 de gaz sec par laquelle le gaz à humidifier provenant du ventilateur 25 pénètre dans le réservoir à eau 1 et une sortie 34 de gaz humidifié par laquelle le gaz humidifié au sein de la cuve 2 ressort du réservoir à eau 1 et est ensuite envoyé vers le patient, via une conduite de gaz flexible par exemple reliée à une interface patient de distribution de gaz, tel un masque respiratoire ou analogue.

L'humidificateur de gaz 24 comprend en outre un élément chauffant 27, telle une résistance électrique ou analogue, et une plaque métallique chauffante 26 qui sont agencés dans le fond 15 du boitier de l'humidificateur 24. L'élément chauffant 27 de l'humidificateur de gaz 24 est commandé par les moyens de commande 20, 22 du ventilateur médical 25 et est par ailleurs en contact avec la plaque métallique 26 de manière à chauffer cette plaque métallique 26 en réponse à un signal de commande de chauffe délivré par les moyens de commande 20, 22 du ventilateur médical 25.

La cuve 2 du réservoir 1 comprend une autre plaque métallique chauffante 6, agencée dans le fond 4 de la cuve 2, qui est elle-même en contact avec la plaque métallique 26 du boitier 1. Les calories générées par l'élément chauffant 27 et transmises à la plaque métallique 26 du boitier 14 passent ensuite à la plaque métallique 6 agencée dans le fond 4 de la cuve 2, puis à l'eau 31 de la cuve 2 de sorte de chauffer cet eau 31.

Plus précisément, la plaque métallique 6 est fixée par collage de manière étanche, comme expliqué ci-après, dans le fond 4 de la cuve 2 du réservoir 1, lequel fond 4 définit avec la paroi périphérique 3 de la cuve 2 du réservoir 1, un volume interne 5 contenant l'eau 31 à chauffer qui sert à humidifier le gaz fourni par le ventilateur médical 25. L'eau 31 contenue dans le réservoir 2 est donc vaporisée par chauffage au contact de la plaque métallique 6 situé en fond 4 de cuve 2.

Les plaques métalliques 6, 26 sont par exemple en aluminium, en alliage d'aluminium, en cuivre, en laiton ou en acier inoxydable, de préférence en acier inoxydable.

Le ventilateur médical 25 est alimenté en courant électrique par une alimentation 17 en courant électrique, par exemple le secteur délivrant un courant ayant une tension entre 110 et 230 V. Le courant fourni par la source de courant électrique 17 est acheminé par un câble électrique venant se raccorder électriquement au ventilateur 25 au niveau d'un connecteur électrique amont 18. Le ventilateur médical 25 est quand à lui raccordé électriquement à l'humidificateur de gaz 24 par un connecteur électrique aval 19 ou analogue. Le courant électrique est ensuite acheminé depuis le connecteur électrique aval 19 jusqu'à l'élément chauffant 27 par un ou plusieurs câbles électriques 29.

Dans les modes de réalisation des Figures 2 à 6, la cuve 2 à eau est parallélépipédique rectangle, c'est-à-dire de section rectangulaire. Elle comprend donc une paroi périphérique 3 formée de 4 panneaux de paroi se faisant face 2 à 2, i.e. parallèles 2 à 2 et perpendiculaires 2 à 2, incluant deux petits panneaux de paroi et deux grands panneaux de paroi, les petits panneaux ayant une largeur inférieure à celle des grands panneaux, la hauteur des petits et des grands panneaux étant (approximativement) égale. De préférence, la cuve 2 à eau est en polymère, par exemple en polypropylène (PP).

A titre indicatif mais non limitatif, les petits panneaux de paroi du réservoir 1 peuvent avoir une largeur I_{R} comprise entre 3 et 8 cm, par exemple entre 4 cm et 6 cm, et les grands panneaux de paroi peuvent avoir une longueur L_{R} comprise entre 10 et 15 cm, par exemple entre 11 et 13 cm. Par ailleurs, la hauteur H_{R} des petits et des grands panneaux peut être comprise entre 9 et 15 cm, par exemple de l'ordre de 10 à 12 cm.

Selon la présente invention, afin d'assurer une solidarisation efficace de la plaque métallique chauffante 6 dans le fond 4 de la cuve 2, ainsi qu'une bonne étanchéité fluidique entre eux, de manière à éviter les fuites d'eau, la plaque métallique chauffante 6 est fixée de manière étanche dans le fond 4 de la cuve 2 au moyen de plusieurs colles 7, 8 déposées entre la plaque chauffante 6 et le fond 4 de la cuve 2.

Selon le type de colle choisi et/ou le mode de réalisation de la cuve 2, on utilise plusieurs colles 7, 8 différentes, c'est-à-dire présentant des caractéristiques différentes l'une de l'autre.

Ainsi, on peut utiliser :
- une première colle 7 choisie parmi les colles de type néoprène, nitrile, polyuréthane, silicone, acrylique, anaérobique, cyanoacrylate, époxy, inorganique, phénolique, polyester ou polysulfure, et
- une deuxième colle 8 de type colle capillaire, par exemple une colle UV à base acrylate ou à base époxy.

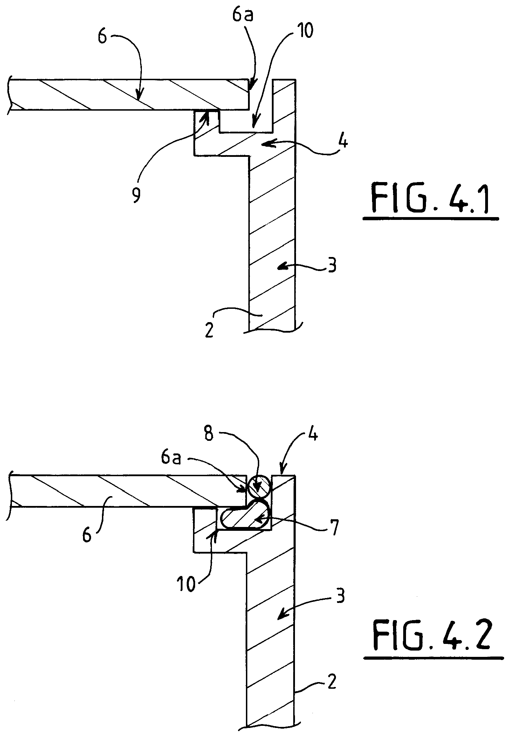

Les Figures 2 à 4 schématisent un premier mode de réalisation d'un réservoir 1 à eau selon l'invention, dans lequel le fond 4 de la cuve 2 à eau comprend un épaulement 9 périphérique s'étendant sur son contour. Cet épaulement 9 comprend par ailleurs une rainure ou gorge périphérique 10 s'étendant aussi sur tout le pourtour du fond 4. La plaque métallique chauffante 6 vient se positionner sur l'épaulement 9 mais hors de la rainure périphérique 10, comme illustré en Figure 4.1.

Afin de solidariser la plaque métallique chauffante 6 au fond 4 situé à l'extrémité inférieure de la paroi périphérique 3 de la cuve 2, au moins une colle 7, 8 est introduite dans la rainure périphérique 10, comme illustré en figure 4.2, de préférence deux colles 7, 8 présentant des propriétés/caractéristiques différentes l'une de l'autre.

D'abord, une première colle 7 est introduite dans le fond de la rainure 10, puis recouverte d'une seconde colle 8 qui vient recouvrir la première colle 7. Celles-ci sont introduites entre le rebord périphérique 6a de la plaque 6 et la paroi de fond 4, comme visible sur les Figures 4.1 et 4.2.

Pour ce faire, on peut utiliser une colle dite « flexible », tel que néoprène, nitrile, polyuréthane ou silicone, ou une colle dite « rigide », tel qu'acrylique, anaérobique, cyanoacrylates, époxy, inorganique, phénolique, polyester, polysulfures. De préférence, on utilise une colle flexible de type silicone pour fixer la plaque métallique chauffante 6 à l'épaulement 9 en fond 4 de cuve 2.

Les deux colles étant différentes, on choisit de préférence une première colle 7 « flexible » et une seconde colle 8 « rigide ».

Dans ce cas, la première colle 7 est déposée au fond de la gorge 10 portée par l'épaulement 9 (cf. Figure 4.2) de manière à assurer l'essentiel de l'étanchéité entre le fond 4 de la cuve 2 et la plaque métallique 6, et à garantir leur solidarisation, comme dans le cas précédent, alors que la deuxième colle 8 est déposée au-dessus de la première colle 7, c'est-à-dire qu'elle forme une couche recouvrant la première colle 7 (cf. Fig. 4.2), de manière à aller remplir et combler les interstices laissés vides par la première colle, interstices entre la plaque métallique 6, la première colle 7 et le fond 4 de la cuve 2 du réservoir 1.

Ainsi, on peut utiliser :
- une première colle 7 de type silicone, par exemple la colle silicone MOMENTIVE™, *Room-Temperature-Vulcanization* (i.e., vulcanisation à température ambiante), et
- une deuxième colle 8 de type colle capillaire (i.e., liquide comme l'eau), par exemple une colle UV à base acrylate ou époxy.

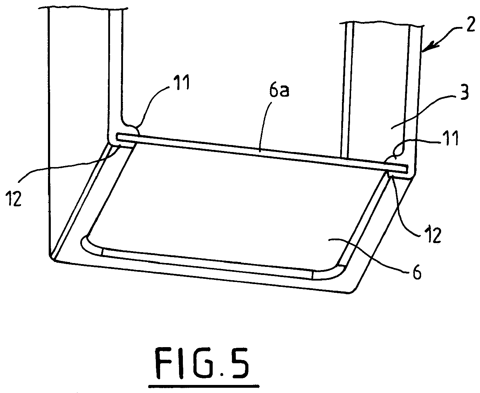

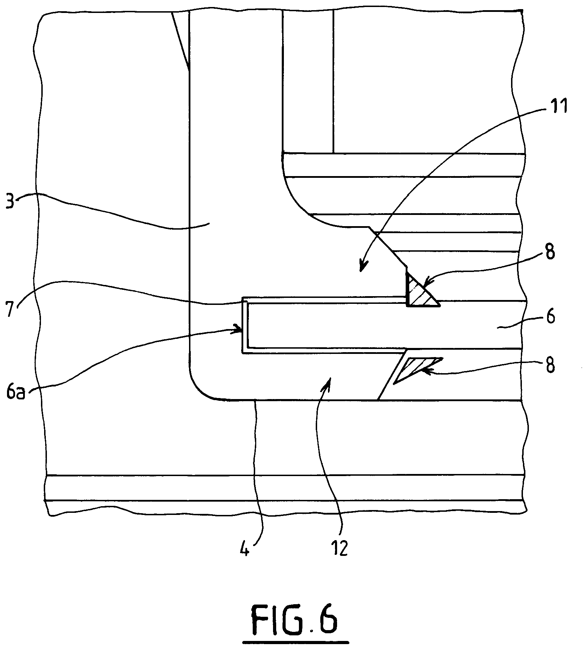

Par ailleurs, les Figures 5 et 6 schématisent un second mode de réalisation d'un réservoir 1 à eau selon l'invention, dans lequel le fond 4 de la cuve 2 à eau comprend deux portions de paroi 11, 12 parallèles l'une à l'autre prenant en sandwich le bord périphérique 6a de la plaque métallique 6, comme illustré en Figures 5 et 6.

De préférence, les deux portions de paroi 11, 12 sont moulées autour du bord périphérique 6a de la plaque métallique chauffante 6.

Là encore, la plaque métallique 6 est maintenue solidaire du fond 4 de la cuve 2 par plusieurs colles 7, 8, de préférence au moins deux colles différentes l'une de l'autre.

Les deux colles 7, 8 différentes que l'on utilise sont :
- une première colle 7 plutôt « flexible » de type silicone, par exemple la colle silicone MOMENTIVE™, comme dans le mode de réalisation précédent, laquelle va assurer l'essentiel de l'étanchéité. Celle-ci est placée entre les deux portions de paroi 11, 12 de fond de la cuve 2 et la plaque métallique 6. Elle assurer une fonction double d'étanchéité et de solidarisation des pièces qui sont de nature différente, à savoir la cuve 2 en plastique et plaque métallique 6.
- et une deuxième colle 8 « rigide », de préférence une colle capillaire, c'est-à-dire liquide comme l'eau, qui va remplir les interstices entre la plaque métallique 6, la première colle 7 et la cuve 2 du réservoir 1, c'est-à-dire les interstices laissés vides par la première colle 7 et ceux entre la plaque métallique 6, la première colle 7 et les deux portions de paroi 11, 12 de fond de la cuve 2, par exemple une colle UV à base acrylate ou epoxy.

La fabrication de la cuve 2 du réservoir 1 à eau des Figures 5 et 6 comprend les étapes principales suivantes, à savoir :
a) une mise en place de la plaque 6 dans un outillage d'injection de la cuve 2 du réservoir 1,
b) une fabrication par moulage du réservoir 1 avec surmoulage la plaque métallique 6 avec prise en sandwich du pourtour de celle-ci par les deux portions de paroi 11, 12 de fond de la cuve 2, et
c) dépôt des colles 7, 8 dans la zone de collage située entre la plaque métallique 6 et les deux portions de paroi 11, 12 de fond de la cuve 2.

Quel que soit le mode de réalisation, les deux colles 7, 8 sont choisies de telle sorte qu'elles assurent un maintien et une étanchéité complémentaires. En d'autres termes, les deux colles 7, 8 sont choisies de telle sorte qu'une fois l'ensemble cuve 2 et plaque 6 assemblé, le réservoir est étanche à l'air et à l'eau, résiste aux contraintes imposées par le poids de l'eau, la pression d'air insufflée par le ventilateur et les manipulations par le patient, et résiste aux dilatations de la plaque 6 et de la cuve 2, et aux opérations de désinfection.

D'une façon générale, un réservoir 1 à cuve 2 à eau selon l'invention est destiné à équiper un humidificateur de gaz 24 d'un ensemble humidificateur 24/ventilateur médical 25 dont le ventilateur 25 est équipé d'une micro-soufflante électrique alimentant l'humidificateur 24 en gaz à humidifier, en particulier en air pressurisé, comme illustré en Figure 1.

## Revendications

1. Réservoir à eau (1) pour humidificateur de gaz comprenant une cuve (2) comprenant une paroi périphérique (3) et un fond (4) délimitant un volume interne (5) destiné à recevoir de l'eau à chauffer, et une plaque métallique chauffante (6) agencée dans le fond (4) de la cuve (2), **caractérisé en ce que** la plaque métallique chauffante (6) est fixée de manière étanche dans le fond (4) de la cuve (2) au moyen d'au moins une première colle (7, 8) déposée entre la plaque chauffante (6) et le fond (4) de la cuve (2), et une deuxième colle (8), différentes l'une de l'autre, où :
- la première colle (7) est choisie parmi les colles de type néoprène, nitrile, polyuréthane, silicone, acrylique, anaérobique, cyanoacrylate, époxy, inorganique, phénolique, polyester ou polysulfure, et est déposée entre le fond (4) de la cuve (2) et la plaque métallique (6) pour assurer une étanchéité entre le fond (4) de la cuve (2) et la plaque métallique (6), et garantir leur solidarisation,
- la deuxième colle (8) est une colle capillaire déposée au-dessus de la première colle (7) et remplissant les interstices entre la plaque métallique (6), la première colle (7) et la cuve (2) du réservoir (1).

2. Réservoir selon la revendication précédente, **caractérisé en ce que** la deuxième colle (8) est une colle UV à base acrylate ou à base époxy.

3. Réservoir selon l'une des revendications précédentes, **caractérisé en ce que** la paroi périphérique (3) est en plastique, de préférence en polypropylène.

4. Réservoir selon l'une des revendications précédentes, **caractérisé en ce que** la plaque métallique chauffante (6) est en acier inoxydable, en aluminium ou en alliage d'aluminium, en cuivre ou en laiton.

5. Réservoir selon l'une des revendications précédentes, **caractérisé en ce que** le fond (4) de la cuve (2) comprend un épaulement (9) comprenant une rainure périphérique (10) destinée à recevoir au moins une partie de la colle de manière fixer la plaque métallique chauffante (6) audit épaulement (9), ladite plaque métallique chauffante (6) étant positionnée sur l'épaulement (9) et hors de la rainure périphérique (10).

6. Réservoir selon la revendication 5, **caractérisé en ce que** la première colle (7) est déposée au fond de la gorge (10) portée par l'épaulement (9).

7. Réservoir selon l'une des revendications précédentes, **caractérisé en ce que** la deuxième colle (8) forme une couche recouvrant la première colle (7).

8. Réservoir selon l'une des revendications 1 à 6, **caractérisé en ce que** le fond (4) de la cuve (2) comprend deux portions de paroi (11, 12) parallèles l'une à l'autre prenant en sandwich le bord périphérique (6a) de ladite plaque métallique chauffante (6), de préférence les deux portions de paroi (11, 12) sont moulées autour du bord périphérique (6a) de ladite plaque métallique chauffante (6).

9. Humidificateur de gaz (24), notamment d'air, comprenant un réservoir (1) à eau selon l'une des revendications précédentes, de préférence un réservoir (1) amovible.

10. Installation de ventilation comprenant un ventilateur médical (25) alimentant en gaz respiratoire un humidificateur de gaz (24) selon la revendication 9.

## Patentansprüche

1. Wasserbehälter (1) für einen Gasbefeuchter, umfassend einen Tank (2), umfassend eine Umfangswand (3) und einen Boden (4), die ein Innenvolumen (5) definieren, das dazu bestimmt ist, zu erwärmenden Wasser aufzunehmen, und eine metallische Heizplatte (6), die im Boden (4) des Tanks (2) angeordnet ist, **dadurch gekennzeichnet, dass** die metallische Heizplatte (6) mittels mindestens eines ersten Klebstoffs (7, 8), der zwischen der Heizplatte (6) und dem Boden (4) des Tanks (2) aufgebracht ist, und eines zweiten Klebstoffs (8), der sich von dem ersten unterscheidet, dicht im Boden (4) des Tanks (2) befestigt ist, wobei:
- der erste Klebstoff (7) unter den Klebstoffen vom Typ Neopren, Nitril, Polyurethan, Silikon, Acryl, anaerob, Cyanacrylat, Epoxid, anorganisch, phenolisch, Polyester oder Polysulfid ausgewählt wird und zwischen dem Boden (4) des Tanks (2) und der metallischen Platte (6) aufgebracht wird, um eine Abdichtung zwischen dem Boden (4) des Tanks (2) und der metallischen Platte (6) zu gewährleisten und ihre feste Verbindung zu garantieren,
- der zweite Klebstoff (8) ein Kapillarklebstoff ist, der über dem ersten Klebstoff (7) aufgebracht ist und die Zwischenräume zwischen der metallischen Platte (6), dem ersten Klebstoff (7) und dem Tank (2) des Behälters (1) ausfüllt.

2. Behälter nach dem vorstehenden Anspruch, **dadurch gekennzeichnet, dass** es sich bei dem zweiten Klebstoff (8) um einen UV-Klebstoff auf Acrylat- oder Epoxidbasis handelt.

3. Behälter nach einem der vorstehenden Ansprüche, **dadurch gekennzeichnet, dass** die Umfangswand (3) aus Kunststoff, bevorzugt Polypropylen, hergestellt ist.

4. Behälter nach einem der vorstehenden Ansprüche, **dadurch gekennzeichnet, dass** die metallische Heizplatte (6) aus rostfreiem Stahl, aus Aluminium oder aus Aluminiumlegierung, aus Kupfer oder aus Messing hergestellt ist.

5. Behälter nach einem der vorstehenden Ansprüche, **dadurch gekennzeichnet, dass** der Boden (4) des Tanks (2) einen Ansatz (9), umfassend eine Umfangsnut (10), umfasst, die dazu bestimmt ist, mindestens einen Teil des Klebstoffs aufzunehmen, sodass die metallische Heizplatte (6) an dem Ansatz (9) befestigt wird, wobei die metallische Heizplatte (6) auf dem Ansatz (9) und außerhalb der Umfangsnut (10) angeordnet ist.

6. Behälter nach Anspruch 5, **dadurch gekennzeichnet, dass** der erste Klebstoff (7) auf dem Boden der von dem Ansatz (9) getragenen Nut (10) aufgebracht ist.

7. Behälter nach einem der vorstehenden Ansprüche, **dadurch gekennzeichnet, dass** der zweite Klebstoff (8) eine Schicht bildet, die den ersten Klebstoff (7) bedeckt.

8. Behälter nach einem der Ansprüche 1 bis 6, **dadurch gekennzeichnet, dass** der Boden (4) des Tanks (2) zwei zueinander parallele Wandabschnitte (11, 12) umfasst, die den Umfangsrand (6a) der metallischen Heizplatte (6) sandwichartig umschließen, wobei bevorzugt die beiden Wandabschnitte (11, 12) um den Umfangsrand (6a) der metallischen Heizplatte (6) herum formgegossen sind.

9. Gasbefeuchter (24), insbesondere für Luft, umfassend einen Wasserbehälter (1) nach einem der vorstehenden Ansprüche, bevorzugt einen abnehmbaren Behälter (1).

10. Beatmungsanlage, umfassend ein medizinisches Beatmungsgerät (25), das einen Gasbefeuchter (24) nach Anspruch 9 mit Atemgas versorgt.

## Claims

1. Water tank (1) for gas humidifier comprising a container (2) comprising a peripheral wall (3) and a bottom (4) delimiting an internal volume (5) intended to receive the water to be heated, and a metal heating plate (6) arranged at the bottom (4) of the container (2), **characterised in that** the metal heating plate (6) is fastened in a sealed manner in the bottom (4) of the container (2) by means of at least one first adhesive (7, 8) deposited between the heating plate (6) and the bottom (4) of the container (2), and a second adhesive (8), different from each other, wherein:
- the first adhesive (7) is selected from among adhesives of the neoprene, nitrile, polyurethane, silicone, acrylic, anaerobic, cyanoacrylate, epoxy, inorganic, phenolic, polyester or polysulphide type, and is deposited between the bottom (4) of the container (2) and the metal plate (6) to provide a seal between the bottom (4) of the container (2) and the metal plate (6), and guarantee the securing thereof,
- the second adhesive (8) is a capillary adhesive deposited above the first adhesive (7) and filling the interstices between the metal plate (6), the first adhesive (7) and the container (2) of the tank (1).

2. Tank according to the preceding claim, **characterised in that** the second adhesive (8) is a UV adhesive with an acrylate base or an epoxy base.

3. Tank according to one of the preceding claims, **characterised in that** the peripheral wall (3) is made of plastic, preferably from polypropylene.

4. Tank according to one of the preceding claims, **characterised in that** the metal heating plate (6) is made of stainless steel, aluminium or aluminium alloy, copper or brass.

5. Tank according to one of the preceding claims, **characterised in that** the bottom (4) of the container (2) comprises a shoulder (9) that comprises a peripheral groove (10) intended to receive at least one portion of the adhesive so as to fasten the metal heating plate (6) to said shoulder (9), said metal heating plate (6) being positioned on the shoulder (9) and outside the peripheral groove (10).

6. Tank according to claim 5, **characterised in that** the first adhesive (7) is deposited at the bottom of the groove (10) carried by the shoulder (9).

7. Tank according to one of the preceding claims, **characterised in that** the second adhesive (8) forms a layer that covers the first adhesive (7).

8. Tank according to one of claims 1 to 6, **characterised in that** the bottom (4) of the container (2) comprises two portions of wall (11, 12) parallel to one another sandwiching the peripheral edge (6a) of said metal heating plate (6), preferably the two portions of wall (11, 12) are moulded around the peripheral edge (6a) of said metal heating plate (6).

9. Gas humidifier (24), in particular of air, comprising a water tank (1) according to one of the preceding claims, preferably a removable tank (1).

10. Ventilation installation comprising a medical ventilator (25) that supplies a gas humidifier (24) with respiratory gas according to claim 9.
